# EUROPÄISCHE PATENTSCHRIFT

(11) **EP 2 084 120 B1**
(45) Veröffentlichungstag und Bekanntmachung des Hinweises auf die Patenterteilung: **23.09.2015**
(21) Anmeldenummer: 07821420.2
(22) Anmeldetag: 17.10.2007
(51) Int. Cl.: C07C 57/04, C07C 57/07, C07C 51/43, C07C 51/235, C07C 51/42, C07C 51/44

(54) **VERFAHREN ZUR TRENNUNG VON IN EINEM PRODUKTGASGEMISCH EINER PARTIELLEN HETEROGEN KATALYSIERTEN GASPHASENOXIDATION EINER C3-VORLÄUFERVERBINDUNG DER ACRYLSÄURE ENTHALTENER ACRYLSÄURE UND BENZOESÄURE**
METHOD OF SEPARATING THE ACRYLIC ACID FROM BENZOIC ACID CONTAINED IN A PRODUCT GAS MIXTURE FROM A HETEROGENEOUSLY CATALYZED GAS PHASE PARTIAL OXIDATION OF A C3-PRECURSOR COMPOUND OF ACRYLIC ACID
PROCEDE DE SEPARATION D'ACIDE BENZOÏQUE ET D'ACIDE ACRYLIQUE CONTENANT UN COMPOSE PRECURSEUR C3 DE L'ACIDE ACRYLIQUE DANS UN MELANGE DE GAZ PRODUIT D'UNE OXYDATION DE PHASE GAZEUSE CATALYSEE HETEROGENE PARTIELLE

(30) Priorität: 19.10.2006 DE 102006049939; 19.10.2006 US 852674 P
(43) Veröffentlichungstag der Anmeldung: 05.08.2009
(73) Patentinhaber: BASF SE, 67056 Ludwigshafen (DE)
(72) Erfinder: HEILEK, Jörg, 69245 Bammental (DE); MÜLLER-ENGEL, Klaus Joachim, 76297 Stutensee (DE); DIETERLE, Martin, 67063 Ludwigshafen (DE); DIEFENBACHER, Armin, 67361 Freisbach (DE)
(86) Internationale Anmeldenummer: PCT/EP2007/061055
(87) Internationale Veröffentlichungsnummer: WO 2008/046840

(56) Entgegenhaltungen:
- EP-A- 0 792 867
- EP-A1- 1 484 308
- EP-A1- 1 484 309
- WO-A-00/05188
- WO-A-01/77056
- WO-A-99/14181

## Beschreibung

Vorliegende Erfindung betrifft ein Verfahren zur Trennung von in einem Produktgasgemisch einer partiellen heterogen katalysierten Gasphasenoxidation einer C₃-Vorläuferverbindung der Acrylsäure neben anderen Produktgasgemischbestandteilen als Haupt- und Nebenprodukt enthaltener Acrylsäure und Benzoesäure, bei dem man die Acrylsäure und die Benzoesäure gemeinsam mit anderen leichter und schwerer als Acrylsäure siedenden Bestandteilen des Produktgasgemisches aus dem Produktgasgemisch in eine flüssige Phase P überführt und aus der dabei resultierenden flüssigen Phase P unter Anwendung wenigstens eines thermischen Trennverfahrens leichter als Benzoesäure und Acrylsäure siedende Bestandteile unter Verbleib einer flüssigen Phase P*, die wenigstens 80 Gew.-% Acrylsäure und, bezogen auf die enthaltene Acrylsäuremenge, wenigstens 0,1 Gew.-‰ Benzoesäure enthält, abtrennt.

Acrylsäure ist ein bedeutendes Monomeres, das als solches und/oder in Form seiner Alkylester zur Erzeugung von im Hygienebereich eingesetzten Polymerisaten (z. B. Wasser superabsorbierenden) Verwendung findet (vgl. z. B. WO 02/055469 und WO 03/078378).

Die Herstellung von Acrylsäure kann z. B. durch heterogen katalysierte partielle Oxidation einer C₃-Vorläuferverbindung (Propylen, Propan und/oder Acrolein) erfolgen (vgl. z. B. EP-A 990636, US-A 5198578, EP-A 1015410, EP-A 1484303, EP-A 1484308, EP-A 1484309 und US-A 2004/0242826).
Als C₃-Vorläuferverbindung wird dabei anwendungstechnisch zweckmäßig in der Regel eine C₃-Vorläuferverbindung von vergleichsweise hoher Reinheit verwendet (vgl. DE-A 10131297). Die Erzeugung von z. B. solchermaßen reinem Roh-Propylen ist jedoch relativ aufwendig und kostspielig und beinhaltet in der Regel verschiedene Reinigungsstufen, um die C₃-Vorläuferverbindung der Acrylsäure hochrein zu isolieren (vgl. DE-A 3521458). Gemäß der DE-A 12246119 und der DE-A 10245585 soll dabei insbesondere so vorgegangen werden, dass das resultierende Reaktionsgasgemisch für die Partialoxidation möglichst keine C₄-Kohlenwasserstoffe als unerwünschte, die Katalysatorperformance beeinträchtigende, Verunreinigungen enthält. Nachteilig an einer solchen Verfahrensweise ist, dass die vorgenannten Reinigungsstufen aufwendig sind, weshalb sie im Sinne einer wirtschaftlichen Roh-C₃-Vorläuferverbindung (z. B. Roh-Propylen) teilweise nur mit vergleichsweise limitierter Trennwirkung angewendet werden.

Sorgfältige eigene Untersuchungen haben nun ergeben, dass dann, wenn die für die heterogen katalysierte Partialoxidation der C₃-Vorläuferverbindung (z. B. des Propylens) verwendete Roh-C₃-Vorläuferverbindung (z. B. das Roh-Propylen) noch Butadien oder Verbindungen, die im Verlauf der Partialoxidation in Butadien gewandelt werden, enthält, als Nebenprodukt der Acrylsäurebildung Benzoesäure gebildet werden kann. Ursächlich ist die Benzoesäurebildung vermutlich auf Diels-Alder Reaktionen des Butadiens mit im Reaktionsgasgemisch der heterogen katalysierten Partialoxidation enthaltenen C₃-Dienophilen wie Propylen, Acrolein und Acrylsäure zurückzuführen, an die sich wahrscheinlich eine heterogen katalysierte Oxydehydrierung des Adduktes zum Aromaten anschließt. Letztere wird vermutlich durch die gleichen Katalysatoren wie die eigentliche Zielgasphasenoxidation katalysiert. Gegebenenfalls kann auch eine geringfügige Verunreinigung der Roh-C₃-Vorläuferver-bindung (z. B. des Roh-Propylens) mit o-Xylol ursächlich für eine Benzoesäurenebenproduktbildung sein. Der Einsatz von Multimetalloxidkatalysatoren mit erhöhter Aktivität (bzw. von Katalysatorbeschickungen, deren volumenspezifische Aktivität in Richtung eines zunehmenden Eduktumsatzes wenigstens einmal zunimmt), eine Mitverwendung von Wasserdampf als inertem Verdünnungsgas sowie ein Überschuss von molekularem Sauerstoff relativ zur partiell zu oxidierenden C₃-Verbindung in den jeweiligen Oxidationsschritten (z. B. Propylen → Acrylsäure) scheint die Benzoesäurenebenproduktbildung ebenso zu fördern, wie erhöhte Reaktionstemperaturen und Umsätze der jeweiligen C₃-Vorläuferverbindung in der jeweiligen Oxidationsstufe (z. B. des Propylens in der ersten Oxidationsstufe einer zweistufigen Partialoxidation von Propylen zu Acrylsäure, oder des Acroleins, in der zweiten Oxidationsstufe einer Partialoxidation von Propylen zu Acrylsäure, oder des Acroleins in einer eigenständigen Acroleinpartialoxidation zu Acrylsäure, oder des Propans, in einer einstufigen Partialoxidation von Propan zu Acrylsäure). Vorgenannte Annahmen werden dadurch gestützt, dass in vielen Fällen nicht Benzoesäure, sondern lediglich Benzaldehyd als Nebenprodukt der Acrylsäurebildung gefunden wird. Umgekehrt tritt Benzoesäure in der Regel in Begleitung von Benzaldehyd als Acrylsäurenebenprodukt auf.

Sowohl Benzaldehyd als auch Benzoesäure sind unerwünschte Begleiter von Acrylsäure. Unerwünscht deshalb, weil sie als vergleichsweise reaktive aromatische Verbindungen nicht völlig unbedenklich sind (dies trifft in entsprechender Weise auf die Ester der Benzoesäure zu). Wird die erzeugte Acrylsäure und/oder deren Alkylester daher zur Herstellung von Polymerisaten eingesetzt, die im Hygienebereich Anwendung finden (z. B. wasserabsorbierende Polymerisate in Babywindeln), ist peinlich darauf zu achten, dass die verwendete Acrylsäure und/oder der verwendete Acrylsäurealkylester an Benzoesäure und Benzaldehyd frei sind.

Es ist allgemein bekannt, Acrylsäure aus dem Produktgasgemisch der heterogen katalysierten partiellen Gasphasenoxidation einer C₃-Vorläuferverbindung der Acrylsäure (z. B. von Propylen) durch eine Kombination unterschiedlicher Trennverfahren abzutrennen. Die im einzelnen angewandte Kombination ist dabei in der Regel abhängig von der Art und Menge der im Produktgasgemisch enthaltenen, von Acrylsäure verschiedenen, Nebenkomponenten sowie von der angestrebten Reinheit der Acrylsäure, die in der Regel an der jeweiligen Verwendung der Acrylsäure ausgerichtet wird.

Wesentlicher Bestandteil solcher Kombinationen unterschiedlicher Trennverfahren bilden normalerweise thermische Trennverfahren. Bei den thermischen Trennverfahren handelt es sich um solche, bei denen in trennwirksame Einbauten enthaltenden Trennkolonnen gasförmige (aufsteigend) und flüssige (absteigend) Stoffströme im Gegenstrom geführt werden, wobei infolge der zwischen den Stoffströmen bestehenden Gradienten ein Wärme- und Stoffaustausch stattfindet, der letztlich die in der Trennkolonne gewünschte Trennung bedingt.

Beispiele für solche thermische Trennverfahren sind die (partielle) Kondensation, die fraktionierende Kondensation (vgl. DE-A 19924532) und die Rektifikation. Die resultierende Trennwirkung beruht hier vor allem auf der Verschiedenheit der Siedepunkte von Acrylsäure und den von Acrylsäure verschiedenen Nebenkomponenten. Ein weiteres Beispiel bildet die Absorption. Die Trennwirkung beruht hier insbesondere auf der unterschiedlichen Löslichkeit von Acrylsäure und den von Acrylsäure verschiedenen Nebenkomponenten in der Absorptionsflüssigkeit. Das Vorgenannte trifft auch auf die thermischen Trennverfahren der Strippung (ein Strippgas nimmt aus einer Flüssigkeit in selbiger gelöst enthaltene Komponenten mit unterschiedlicher Affinität auf) und der Desorption (der Umkehrprozess zur Absorption; das in der Flüssigphase Gelöste wird durch Partialdruckerniedrigung abgetrennt) zu. Der Begriff der thermischen Trennverfahren umfasst aber auch die azeotrope Destillation bzw. Rektifikationen (sie nützen die unterschiedlich ausgeprägte Neigung von Acrylsäure und den Nebenkomponenten (den von Acrylsäure verschiedenen Bestandteilen im Reaktionsgasgemisch der Partialoxidation) zur Ausbildung von Azeotropen mit zugesetzten azeotropen Schleppern aus).

Der Siedepunktunterschied zwischen Acrylsäure (141 °C bei 1 atm) und Benzaldehyd (178,1 °C bei 1 atm) ist in der Regel nicht ausreichend, um das Benzaldehyd mit angemessenem Aufwand unter Anwendung der vorgenannten thermischen Trennverfahren quantitativ von Acrylsäure abzutrennen. Letzteres wird normalerweise vielmehr erst dann erreicht, wenn man zusätzlich die unterschiedlich ausgeprägte Reaktivität von Benzaldehyd und Acrylsäure mit sogenannten Aldehydfängern (WO 03/014172) wie z. B. Anminoguanidinhydrogencarbonat ausnützt. Durch die Reaktion des Aldehyds mit dem Aldehydfänger entsteht eine neue Nebenkomponente, deren Siedepunkt wesentlich stärker vom Siedepunkt der Acrylsäure abweicht, als dies im Fall des Aldehyds selbst der Fall ist, so dass nach erfolgter Umsetzung mit dem Aldehydfänger eine quantitative Abtrennung auf rektifikativem Weg in einfacher Weise erreicht werden kann. Alternativ dazu ist es aus der EP-A 1159249 bekannt, im Produktgasgemisch der Partialoxidation enthaltenen Benzaldehyd und Acrylsäure durch die Kombination aus einem thermischen Trennverfahren (fraktionierende Kondensation) und einer Kristallisation (Suspensionskristallisation) voneinander zu trennen (vgl. auch DE-A 10247240).

Im Unterschied zum Vorgenannten ist der Siedepunktsunterschied zwischen Acrylsäure (141 °C bei 1 atm) und Benzoesäure (250 °C bei 1 atm) so groß, dass bei Anwendung eines thermischen Trennverfahrens, dessen Trennwirkung auf den unterschiedlichen Siedepunkten der Gemischbestandteile fußt, normalerweise eine quantitative Trennung von Acrylsäure und Benzoesäure einhergeht (vgl. DE-A 10336386, DE-A 19740252, DE-A 10247240) (die Abtrennung weiterer Nebenkomponenten erfolgt dann im Anschluss an die Benzoesäureabtrennung häufig kristalliativ). Nachteilig an einer solchen thermischen Trennung ist jedoch, dass die die Acrylsäure angereichert enthaltende Fraktion dabei aus der trennwirksame Einbauten enthaltenden Trennkolonne oberhalb des Zulaufs des aufzutrennenden Gemischs in die Trennkolonne entnommen werden muss. Dies ist jedoch ein thermisch sehr aufwendiges Unterfangen, da ein Verdampfen der Acrylsäuregesamtmenge energetisch vergleichsweise aufwendig ist (hoher Siedepunkt, hohe Verdampfungsenthalpie, Mehrfachverdampfung infolge Rücklauf).

Die Aufgabe der vorliegenden Erfindung bestand daher darin, ein Verfahren zur Trennung von in einem Produktgasgemisch einer partiellen heterogen katalysierten Gasphasenoxidation einer C₃-Vorläuferverbindung der Acrylsäure neben anderen Produktgasgemischbestandteilen als Haupt- und Nebenprodukt enthaltener Acrylsäure und Benzoesäure zur Verfügung zustellen, das der vorgenannten einergieintensiven Trennweise nicht bedarf.

Demgemäss wird ein Verfahren zur Trennung von in einem Produktgasgemisch einer partiellen heterogen katalysierten Gasphasenoxidation einer C₃-Vorläuferverbindung der Acrylsäure neben anderen Produktgasgemischbestandteilen als Haupt- und Nebenprodukt enthaltener Acrylsäure und Benzoesäure, bei dem man die Acrylsäure und die Benzoesäure gemeinsam mit anderen leichter und schwerer als Acrylsäure siedenden Bestandteilen des Produktgasgemisches aus dem Produktgasgemisch in eine flüssige Phase P überführt und von der dabei resultierenden flüssigen Phase P unter Anwendung wenigstens eines thermischen Trennverfahrens leichter als Benzoesäure und Acrylsäure siedende (bezogen auf den Siedepunkt der reinen Stoffe bei 1 atm) Bestandteile (aber keine Benzoesäure; "keine" heißt hier weniger als 10 Gew.-%, vorzugsweise weniger als 5 Gew.-%, noch besser weniger als 3 Gew.-% oder weniger als 2 Gew.-% bzw. weniger als 1 Gew.-%, und am allerbesten weniger als 0,75 Gew.-%, oder weniger als 0,1 Gew.-% bzw. 0 Gew.-%, jeweils bezogen auf den Benzoesäuregehalt der flüssigen Phase P) unter Verbleib einer flüssigen Phase P*, die wenigstens 80 Gew.-% Acrylsäure und, bezogen auf die enthaltene Acrylsäuremenge, wenigstens 0,1 Gew.-‰ (häufig wenigstens 0,2 Gew.- ‰, oder wenigstens 0,3 Gew.- ‰, oder wenigstens 0,4 Gew.- ‰, oder wenigstens 0,5 Gew.- ‰, oder wenigstens 0,75 Gew.- ‰, oder wenigstens 1 Gew.-‰) Benzoesäure enthält, abtrennt, zur Verfügung gestellt, das dadurch gekennzeichnet ist, dass die Trennung der Benzoesäure von der Acrylsäure kristallisativ aus der flüssigen Phase P* heraus erfolgt, wobei sich die Acrylsäure im gebildeten Kristallisat und die Benzoesäure in der verbliebenen Mutterlauge anreichert.

Das erfindungsgemäße Verfahren fußt auf dem überraschenden Befund, dass der mit der Kristallisation einhergehende Abreicherungskoeffizient A^{BZS} (prozessbezogen) regelmäßig ≥ 15 beträgt. Unter dem Abreicherungskoeffizienten A^{BZS} wird dabei generell das Mengenverhältnis von in der Mutterlauge verbliebener Benzoesäure zu im Kristallisat verbliebener Benzoesäure verstanden (jeweils ausgedrückt als Gew.-% bezogen auf die Gesamtmenge an Mutterlauge bzw. die Gesamtmenge an Kristallisat). Eine Kristallisat-/Mutterlaugen-abtrennung zu mehr als 90 Gew.-%, vorzugsweise zu mehr als 95 Gew.-%, oder 97 oder 98, oder 99 Gew.-% ihrer Gesamtmenge ist zur Bestimmung von A^{BZS} in der Regel ausreichend (der Einfluß der Restfeuchte auf dem Kristallisat ist in der Regel vernachlässigbar). Ein A^{BZS} ≥ 15 belegt, dass bei der Ausbildung des Acrylsäurekristalls im wesentlichen kein Einbau der Benzoesäure in den Kristall erfolgt. Darauf fußt die ausgeprägte Effizienz der erfindungsgemäßen Verfahrensweise.

Im Fall von Diacrylsäure (A^{DA}), Essigsäure (A^{ES}) und Propionsäure (A^{PS}) als Acrylsäurenebenkomponenten liegt der entsprechende Abreicherungskoeffizient üblicherweise bei Werten ≤ 10. D. h., sie werden in die Acrylsäurekristalle mit eingebaut und sind aus diesen Kristallen z. B. durch geeignetes Waschen derselben nur schwer zu extrahieren.

D. h., eine kristallisative Abtrennung dieser Verunreinigungen von Acrylsäure erfordert in der Regel die Anwendung wenig effizienter und investitionsintensiver mehrstufiger Kristallisationsverfahren, wie sie z. B. in der EP-A 616998 in Form einer mehrstufigen Kombination von dynamischer und statischer Kristallisation empfohlen wird und dort neben der Mehrstufigkeit wenigstens eines dynamischen und wenigstens eines statischen Kristallisators bedarf. Allenfalls unter besonderen Rahmenbedingungen der Kristallisation (vgl. insbesondere WO 03/078378 und WO 01/77056) bilden sich Acrylsäurekristallformen aus, aus denen durch nachfolgendes Waschen mit reiner Acrylsäureschmelze Essigsäure und Propionsäure vergleichsweise gut entfernt werden können. Je größer A^{BZS} ist, desto attraktiver ist eine kristallisative Abtrennung der Benzoesäure von Acrylsäure.

Die erfindungsgemäße Verfahrensweise eröffnet mit den vorgenannten experimentellen Befunden die Möglichkeit, auf dem Weg zu superabsorbertauglicher Acrylsäure die einer solchen Verwendung im Weg stehende Benzoesäureverunreinigung der flüssigen Phase P* in einer Kristallisationsstufe mit nachfolgender Waschung des Kristallisats mit vorgereinigter Acrylsäureschmelze im wesentlichen quantitativ abzutrennen.

Der Wortlaut "Acrylsäure als Hauptprodukt" und "Benzoesäure als Nebenprodukt" soll in dieser Schrift lediglich bedeuten, dass das Produktgasgemisch der Partialoxidation wesentlich mehr Acrylsäure als Benzoesäure enthält. D.h., Acrylsäure ist das erwünschte Zielprodukt der Partialoxidation, während Benzoesäure ein im Kern unerwünschtes Nebenprodukt derselben ist.

Das erfindungsgemäße Verfahren ist insbesondere auf solche flüssigen Phasen P* anwendbar, deren Acrylsäuregehalt wenigstens 85 Gew.-%, oder wenigstens 90 Gew.-%, oder wenigstens 95 Gew.-%, oder wenigstens 96 Gew.-%, oder wenigstens 97 Gew.-%, oder wenigstens 98 Gew.-%, oder wenigstens 99 Gew.-%, oder wenigstens 99,5 Gew.-% oder mehr beträgt.

Dabei kann bei allen vorgenannten (selbstverständlich auch bei einem Gehalt von 80 Gew.- % an Acyrylsäure in der flüssigen Phase P*) Acrylsäuregehalten der flüssigen Phase P*, der auf die enthaltene Acrylsäuremenge bezogene Gehalt an Benzoesäure z.B. jeweils 0,1 bis 20 Gew.-‰, oder 0,2 bis 15 Gew.-‰, oder 0,3 bis 10 Gew.-‰, oder 0,4 bis 8 Gew.- ‰, oder 0,5 bis 6 Gew.- ‰, oder 0,75 bis 4 Gew.- ‰, oder 1 bis 3 Gew.- ‰ betragen. In der Regel ist der Benzoesäuregehalt der flüssigen Phase P*, bezogen auf darin enthaltene Acrylsäure, nicht größer als 100 Gew.- ‰, häufig nicht größer als 75 Gew.- ‰, oder nicht größer als 50 Gew.- ‰. Die Anwendung des erfindungsgemäßen Verfahrens ist ferner insbesondere dann vorteilhaft, wenn der Gehalt der flüssigen Phase P* an Benzoesäure (von 0 verschieden ist und) wenigstens 50 Gew.-%, bzw. wenigstens 75 Gew.-%, oder wenigstens 100 Gew.-%, oder wenigstens 125 Gew.-%, oder wenigstens 150 Gew.-%, oder wenigstens 175 Gew.-%, oder wenigstens 200 Gew.-%, oder wenigstens 250 Gew.-%, oder wenigstens 300 Gew.-%, oder wenigstens 400 Gew.-%, oder wenigstens 500 Gew.-% der in der flüssigen Phase P* enthaltenen Gewichtsmenge an Benzaldehyd beträgt. Selbstverständlich kann die erfindungsgemäß zu behandelnde flüssige Phase P* auch kein Benzaldehyd enthalten.

Von besonderer Bedeutung ist das erfindungsgemäße Verfahren dann, wenn man es so ausübt, dass bei der kristallisativen Abtrennung (aus der flüssigen Phase P* heraus) verbliebene Mutterlauge wenigstens teilweise in wenigstens eines der zur Erzeugung der flüssigen Phase P* aus der flüssigen Phase P angewandten thermischen Trennverfahren und/oder in wenigstens eines der zur gemeinsamen Überführung der im Produktgasgemisch der Gasphasenpartialoxidation enthaltenen Acrylsäure und Benzoesäure in die flüssige Phase P angewandten Verfahren rückgeführt (normalerweise unterhalb des Kopfes der verwendeten trennwirksame Einbauten enthaltenden Trennkolonne) wird.

Die Grundstruktur einer solchen kombinierten Anwendung des scharfen Trennverfahrens der Kristallisation und von unscharfen Trennverfahren zur Erzeugung der flüssigen Phase P* aus dem Produktgasgemisch der heterogen katalysierten partiellen Gasphasenoxidation der wenigstens einen C₃-Vorläuferverbindung ist aus der DE-A 19606877 bekannt.

Ein unscharfes Trennverfahren ist dabei als ein Trennverfahren definiert, bei dem die Zusammensetzung der sich bei Anwendung des Trennverfahrens bildenden, Zielprodukt angereichert enthaltenden, Phase von der Zusammensetzung des zu trennenden Gemischs in ausgeprägter Weise abhängig ist, während die erfindungsgemäße kristallisative Behandlung insofern ein scharfes Trennverfahren ist, als die Zusammensetzung der sich bildenden Acrylsäurekristalle weitgehend unabhängig (im Idealfall besteht völlige Unabhängigkeit) von der Zusammensetzung der flüssigen Phase P* ist. D.h., beim scharfen Trennverfahren der Kristallisation ist aus thermodynamischer Sicht eine einzige Gleichgewichtseinstellung ausreichend, um die gewünschte Trennwirkung zu erzielen, während bei unscharfen Trennverfahren aus thermodynamischer Sicht mehrere aufeinanderfolgende Gleichgewichtseinstellungen durchlaufen werden müssen (Stichwort: Mc Cabe Thiele Trennstufen), um eine signifikante Trennwirkung zu bewirken.

Das erfindungsgemäße Verfahren ist im Fall einer solchen Kombination eines scharfen und wenigstens eines unscharfen Trennverfahrens insofern von erhöhter Bedeutung, als sich beim kontinuierlichen Betrieb einer solchen Verfahrensweise die Benzoesäure in der erfindungsgemäß zu behandelnden flüssigen Phase P* durch die Mutterlaugerückführung aufpegelt, da die Mutterlauge die Benzoesäure angereichert enthält. D.h., auch vergleichsweise geringe Benzoesäuregehalte im Produktgasgemisch der partiellen heterogen katalysierten Gasphasenoxidation können sich so zu einem ernsten Problem auswachsen, das nur dadurch vernünftig zu bewältigen bleibt, dass gemäß erfindungsgemäßem Befund A^{BZS} (prozessbezogen) regelmäßig ≥ 15 beträgt. Im Fall von Abreicherungskoeffizienten A^{BZS} < 15 wäre eine wie beschrieben auszuübende Verfahrensweise äußerst ineffizient. Die insbesondere großtechnisch erforderliche Effizienz erlangt sie nur dadurch, dass A^{BZS} beim erfindungsgemäßen Verfahren regelmäßig ≥ 15 beträgt.

Der eben genannte Sachverhalt ist aber auch dann relevant, wenn beim erfindungsgemäßen Verfahren anfallende Mutterlaugen zum Zweck der Ausbeutesteigerung weiter kristallisiert werden, oder wenn in den unscharfen Trennverfahren anfallende, Benzoesäure enthaltende Nebenstoffströme zur Ausbeutesteigerung ebenfalls kristallisativ behandelt werden.

Die erfindungsgemäße kristallisative Behandlung der flüssigen Phase P* unterliegt grundsätzlich keiner Beschränkung, einschließlich den Verfahren zur Abtrennung der Mutterlauge vom Kristallisat (alle in den zu den möglichen anwendbaren Kristallisationsverfahren zitieren Schriften des Standes der Technik ausgeführten Mutterlauge/Kristallisat-Abtrennungsverfahren sind anwendbar).

D.h., sie kann einstufig, oder mehrstufig, kontinuierlich oder diskontinuierlich durchgeführt werden. Insbesondere kann sie auch als fraktionierte Kristallisation durchgeführt werden. Üblicherweise werden bei einer fraktionierten Kristallisation alle Stufen, die ein Acrylsäurekristallisat erzeugen, das (insbesondere an Benzoesäure) reiner als die zugeführte flüssige Phase P* ist, Reinigungsstufen genannt und alle anderen Stufen Abtriebsstufen. Zweckmäßig werden nun mehrstufige Verfahren nach dem Gegenstromprinzip betrieben, bei dem nach der Kristallisation in jeder Stufe das Kristallisat von der Mutterlauge abgetrennt und dieses Kristallisat der jeweiligen Stufe mit dem nächsthöheren Reinheitsgrad zugeführt wird, während der Kristallisationsrückstand der jeweiligen Stufe mit dem nächstniedrigen Reinheitsgrad zugeführt wird.

In der Regel liegt die Temperatur der flüssigen Phase P* während der kristallisativen Abtrennung zwischen -25°C und +14°C, insbesondere zwischen +12 °C und -5 °C.

Beispielsweise kann das erfindungsgemäße Verfahren als Schichtkristallisation ausgeführt sein (vgl. DE-OS 2606364, EP-A 616998, EP-A 648520 und EP-A 776875). Dabei wird das Kristallisat in Form zusammenhängender, fest anhaftender Schichten ausgefroren. Die Trennung des abgeschiedenen Kristallisats von der verbliebenen Restschmelze (die Mutterlauge) erfolgt durch einfaches Abfließen der Restschmelze. Prinzipiell unterscheidet man zwischen "statischen" und "dynamischen" Schichtkristallisationsverfahren. Kennzeichnend für die dynamische Schichtkristallisation von flüssigen Phasen P* ist eine erzwungene Konvektion der flüssigen Phase P*. Diese kann durch Umpumpen der flüssigen Phase P* durch volldurchströmte Rohre, durch Aufgabe der flüssigen Phase P* als Rieselfilm (z.B. gemäß EP-A 616998) oder durch Einleiten von Inertgas in eine flüssige Phase P* oder durch Pulsieren erfolgen.

Bei den statischen Verfahren ruht die flüssige Phase P* (z.B. in Rohrbündel- oder Plattenwärmetauschern) und scheidet sich schichtförmig durch langsame Temperatursenkung auf der Sekundärseite ab. Danach wird die Restschmelze (Mutterlauge) abgelassen, durch langsame Temperaturerhöhung stärker verunreinigte Fraktionen aus der Kristallschicht abgeschwitzt und nachfolgend das Reinprodukt abgeschmolzen (vgl. WO 01/77056).

Erfindungsgemäß bevorzugt wird der erfindungsgemäße Kristallisationsschritt im Fall aller in dieser Schrift beschriebenen flüssigen Phasen P* jedoch gemäß der Lehre der WO 01/77056, der WO 02/055469 und der WO 03/078378 als Suspensionskristallisation ausgeführt.

Dabei wird in der Regel durch Kühlung der flüssigen Phase P* eine Acrylsäurekristalle suspendiert enthaltende Kristallsuspension erzeugt, wobei die Acrylsäurekristalle einen geringeren und die verbleibende Restschmelze (Mutterlauge) einen höheren Benzoesäuregehalt (relativ bezogen auf die jeweilige Gesamtmenge) aufweist, als die zu reinigende flüssige Phase P*. Dabei können die Acrylsäurekristalle unmittelbar in Suspension befindlich wachsen und/oder sich als Schicht auf einer gekühlten Wand abscheiden, von der sie anschließend abgekratzt und in der Restschmelze (Mutterlauge) resuspendiert werden.

Alle in der WO 01/77056, WO 02/055469, WO 03/078378 sowie in Research Disclosure Database Number 496005 (published in August 2005) aufgeführten Suspensionskristaller und Suspensionskristallisationsverfahren kommen erfindungsgemäß in Betracht. In der Regel weist die dabei erzeugte Acrylsäurekristallisatsuspension einen Feststoffgehalt von 20 bis 40 Gew.-% auf.

Weiterhin kommen alle in den vorgenannten WO-Veröffentlichungen genannten Verfahren zur Trennung von gebildetem Suspensionskristallisat und verbliebener Mutterlauge in Betracht (z.B. mechanische Trennverfahren wie Zentrifugieren). Erfindungsgemäß bevorzugt erfolgt die Trennung in einer Waschkolonne. Bevorzugt handelt es sich dabei um eine Waschkolonne mit erzwungenem Transport der abgeschiedenen Acrylsäurekristalle. Der Kristallvolumenanteil im Kristallbett erreicht dabei in der Regel Werte > 0,5. Im Regelfall wird die Waschkolonne bei Werten von 0,6 bis 0,75 betrieben. Als Waschflüssigkeit wird mit Vorteil die Schmelze von in der Waschkolonne vorab gereinigten (abgetrennten) Acrylsäurekristallen verwendet. Die Wäsche erfolgt normalerweise im Gegenstrom. Damit umfasst das erfindungsgemäße Verfahren insbesondere Verfahren, welche folgende Verfahrensschritte umfassen:
a) Auskristallisieren von Acrylsäure aus einer flüssigen Phase P*,
b) Trennen des Acrylsäurekristallisats von der verbliebenen Mutterlauge (Restschmelze, flüssigen Restphase),
c) wenigstens teilweises Aufschmelzen des abgetrennten Acrylsäurekristallisats und
d) wenigstens teilweises Rückführen des aufgeschmolzenen Acrylsäurekristallisats zum Schritt b) und/oder zum Schritt a).

Bevorzugt erfolgt dabei der Schritt b) durch Gegenstromwäsche mit in den Schritt b) rückgeführtem aufgeschmolzenem zuvor abgetrenntem Acrylsäurekristallisat.

Erfindungsgemäß vorteilhaft (aber nicht in notwendiger Weise) enthält die flüssige Phase P* bei Anwendung des erfindungsgemäßen Verfahrens Wasser, da eine Ausbildung von Acrylsäurekristallisat im Beisein von Wasser gemäß der Lehre der WO 01/77056 und der WO 03/078378 eine für die nachfolgende Trennung des Kristallisats von verbliebener Mutterlauge besonders günstige Kristallform bedingt. Dies gilt insbesondere dann, wenn die Kristallisation als Suspensionskristallisation ausgeführt wird, und noch mehr, wenn die nachfolgende Mutterlaugeabtrennung in einer Waschkolonne ausgeführt wird, und noch mehr, wenn dabei als Waschflüssigkeit die Schmelze von in der Waschkolonne bereits gereinigtem Acrylsäurekristallisat verwendet wird.

D.h., das erfindungsgemäße Verfahren umfasst insbesondere Verfahren, bei denen man die kristallisativ zu behandelnde flüssige Phase P* unter Einwirkung von Kälte in eine aus Acrylsäurekristallisat und flüssiger Restphase (Restschmelze) bestehende Kristallisatsuspension überführt, wobei der Gewichtsanteil des Acrylsäurekristallisats an Benzoesäure kleiner und der Gewichtsanteil der flüssigen Restphase (der Mutterlauge) an Benzoesäure größer als der Benzoesäuregewichtsanteil der flüssigen Phase P* ist, von der Kristallisatsuspension gegebenenfalls einen Teil der verbliebenen Mutterlauge mechanisch abtrennt, und das Acrylsäurekristallisat in einer Waschkolonne von verbliebener Mutterlauge mit der Maßgabe befreit, dass
a) die flüssige Phase P* bezogen auf die darin enthaltene Acrylsäure wenigstens 50 Gew.ppm bzw. 0,20 bis 20, oft bis 15 oder bis 10 Gew.-% an Wasser enthält, und
b) als Waschflüssigkeit die Schmelze von in der Waschkolonne gereinigtem Acrylsäurekristallisat verwendet wird.

Weiterhin ist es erfindungsgemäß vorteilhaft, wenn der Wassergehalt der flüssigen Phase P* bei vorstehend beschriebenen Verfahrensweisen (bzw. ganz generell bei Anwendung des erfindungsgemäßen Verfahrens), bezogen auf in der flüssigen Phase P* enthaltene Acrylsäure, 0,2 bzw. 0,4 bis 8, bzw. bis 10, bzw. bis 20 Gew.-% beträgt.

Alles Vorgenannte gilt außerdem vor allem dann, wenn die Waschkolonne eine Waschkolonne mit erzwungenem Transport der Acrylsäurekristalle ist, und dies vor allem dann, wenn es eine hydraulische oder eine mechanische Waschkolonne gemäß der WO 01/77056 ist und sie wie dort ausgeführt betrieben wird.

Alles Vorgenannte gilt außerdem vor allem dann, wenn die Waschkolonne gemäß den Lehren der WO 03/041832 sowie der WO 03/041833 ausgeführt ist und betrieben wird.

Grundsätzlich kann die heterogen katalysierte partielle Gasphasenoxidation vorab der Anwendung des erfindungsgemäßen Trennverfahrens so durchgeführt werden, wie es aus dem Stand der Technik bekannt ist (sieht man davon ab, dass die zur Erzeugung des Reaktionsgasausgangsgemischs verwendete Roh-C₃-Vorläuferverbindung einen geringeren Reinheitsgrad aufweisen kann).

D.h., beispielsweise kann sie so ausgeführt werden, wie es die DE-A 10351269, die DE-A 10245585, die Deutsche Schrift 1020050227988, die EP-A 1695954, die DE-A 10351269, die EP-A 990636, die EP-A 1106598, die DE-A102005010111, die DE-A 1020050130399, die DE-A 102004025445, die DE-A 102004021764, die DE-A 10338529, die DE-A 10337788, die DE-A 10360396, die DE-A 10316465, die DE-A 10313210, die DE-A 10313214, die DE-A 10313213, die DE-A 10313212, die Deutsche Schrift 102005062026.4, die DE-A 10313211, die DE-A 10313208 und die DE-A 10313209 sowie der in diesen Schriften beschriebene Stand der Technik beschreiben.

Im besonderen ist das erfindungsgemäße Verfahren auf das Produktgasgemisch einer zweistufigen heterogen katalysierten partiellen Gasphasenoxidation von Propylen zu Acrylsäure anwendbar. In der ersten Reaktionsstufe wird das Propylen im wesentlichen zu Acrolein partialoxidiert und in der zweiten Reaktionsstufe wird das in der ersten Reaktionsstufe erzeugte Acrolein zu Acrylsäure partialoxidiert. Üblicherweise wird das Acrolein dabei als Bestandteil des Produktgasgemischs der ersten Reaktionsstufe, gegebenenfalls ergänzt durch molekularen Sauerstoff oder durch ein Gemisch aus molekularem Sauerstoff und Inertgas, der zweiten Reaktionsstufe zugeführt.

Das erfindungsgemäße Verfahren ist vor allem dann relevant, wenn der Propylenumsatz U^{P} in der Propylenpartialoxidation ≥ 91 mol-%, bzw. ≥ 92 mol-%, oder ≥ 93 mol-%, oder ≥ 94 mol-%, bzw. ≥ 95 mol-%, oder ≥ 96 mol-%, oder ≥ 97 mol-%, oder ≥ 98 mol-%, oder ≥ 99 mol-% beträgt.

Das erfindungsgemäße Verfahren ist ferner vor allem dann relevant, wenn der Acroleinumsatz U^{A} in der Acroleinpartialoxidation ≥ 96 mol-%, bzw. ≥ 97 mol-%, oder ≥ 98 mol-%, oder ≥ 98,5 mol-%, oder ≥ 99 mol-%, bzw. ≥ 99,5 mol-%, oder ≥ 99,8 mol-% oder mehr beträgt.

Dies ist dadurch bedingt, dass vorgenannte Umsätze (sie beziehen sich stets auf den einmaligen Durchgang des Reaktionsgasgemischs durch das Katalysatorbett) bei Verwendung desselben Katalysatorsystems normalerweise dann erzielt werden, wenn die Reaktionstemperatur in der jeweiligen Reaktionsstufe erhöht gewählt und/oder eine Katalysatorbeschickung mit Katalysatoren, die eine erhöhte Aktivität aufweisen, verwendet wird. Beides fördert die Benzoesäurenebenproduktbildung.

Das Vorgenannte gilt insbesondere im Fall der bereits angesprochenen zweistufigen Partialoxidation von Propylen zu Acrylsäure.

Das Katalysatorbett für die heterogen katalysierte Partialoxidation der C₃-Vorläuferverbindung wird in der Regel ein Festbett sein. Besonders aktive (Festbett)katalysatoren für die Propylenpartialoxidation sind solche, deren Aktivmasse eine Multimetalloxidmasse ist, deren spezifische Oberfläche 0,1 bis 120 m²/g, bzw. 0,2 bis 50 m²/g, bzw. 1 bis 20 m²/g, oder 2 bis 10 m²/g beträgt.

Ferner ist es für die (Festbett)katalysatoren der Propylenpartialoxidation zu Acrolein ihre Aktivität betreffend günstig, wenn der numerisch häufigste Porendurchmesser ihrer Multimetalloxidaktivmasse 0,1 bis 1 µm beträgt.

Erhöhte Aktivität der (Festbett)katalysatoren für die Partialoxidation von Propylen zu Acrolein liegt demnach insbesondere dann vor, wenn bei deren Multimetalloxidaktivmasse vorgenannter numerisch häufigster Porendurchmesser und eine der vorgenannten spezifischen Oberflächen kombiniert vorliegen.

Das Porengesamtvolumen der vorgenannten Multimetalloxidaktivmassen(katalysatoren) mit erhöhter Aktivität beträgt dabei vorteilhaft gleichzeitig 0,1 bis 1,00 ml/g, meist 0,10 bis 0,80 ml/g, bzw. 0,20 bis 0,40 ml/g.

Häufig ist es bereits ausreichend, wenn die Katalysatoren für die heterogen katalysierte Partialoxidation von Propylen zu Acrolein vorgenannte Katalysatoren umfassen.

Besonders aktive (Festbett)katalysatoren für die Acroleinpartialoxidation (insbesondere als zweite Reaktionsstufe einer zweistufigen Propylenpartialoxidation zu Acrylsäure) sind solche, deren Aktivmasse eine Multimetalloxidmasse ist, deren spezifische Oberfläche 0,1 bis 150 m²/g, bzw. 0,2 bis 50 m²/g, bzw. 1 bis 20 m²/g oder 2 bis 10 m²/g beträgt.

Ferner ist es für die (Festbett)Katalysatoren der Acroleinpartialoxidation zu Acrylsäure ihre Aktivität betreffend günstig, wenn der numerisch häufigste Porendurchmesser ihrer Multimetalloxidaktivmasse 0,1 bis 1 µm beträgt.

Erhöhte Aktivität der (Festbett)Katalysatoren für die Partialoxidation von Acrolein zu Acrylsäure liegt demnach insbesondere dann vor, wenn bei deren Multimetalloxidaktivmasse vorgenannter numerisch häufigster Porendurchmesser und eine der vorgenannten spezifischen Oberflächen kombiniert vorliegen.

Das Porengesamtvolumen der vorgenannten Multimetalloxidaktivmassen(katalysatoren) mit erhöhter Aktivität beträgt dabei vorteilhaft gleichzeitig 0,1 bis 0,90 ml/g, meist 0,20 bis 0,80 ml/g, bzw. 0,30 bis 0,70 ml/g.

Häufig ist es bereits ausreichend, wenn die Katalysatoren für die heterogen katalysierte Partialoxidation von Acrolein zu Acrylsäure vorgenannte Katalysatoren umfassen. Prinzipiell kann bei beiden vorstehend ausgeführten Partialoxidationen die volumenspezifische Aktivität des Katalysatorbetts (insbesondere Katalysatorfestbetts) in Strömungsrichtung des Reaktionsgasgemischs über die Länge des Strömungsweges konstant sein. Besondere Relevanz für das erfindungsgemäße Verfahren erwächst aber dann, wenn diese volumenspezifische Aktivität des Katalysator(fest)betts in Strömungsrichtung des Reaktionsgasgemischs im jeweiligen Fall wenigstens einmal (kontinuierlich, oder abrupt, oder stufenförmig) zunimmt. In allen vorgenannten Fällen ist es ferner von Vorteil, wenn sich die Aktivmassenzusammensetzung über die Länge des Strömungsweges nicht ändert.

Die vorgenannten Katalysatoren können sowohl Schalenkatalysatoren als auch Vollkatalysatoren sein. Die Geometrie der Katalysatoren kann im Prinzip beliebig sein. Sowohl Kugeln, Polygone, Vollzylinder als auch Ringe kommen in Betracht. Die Längstausdehnung der Katalysatorformkörper beträgt zweckmäßig 1 bis 10 mm, oder 2 bis 8 mm. Darunter versteht man die längste direkte Verbindung zweier auf der Katalysatorformkörperoberfläche befindlicher Punkte. Die volumenspezifische Aktivität des Katalysatorfestbetts lässt sich durch entsprechendes Verdünnen der Katalysatoren mit sich im wesentlichen inert verhaltenden Formkörpern (Verdünnungsformkörpern) bewirken. Deren Geometrie kann jener der Katalysatorformkörper im wesentlichen entsprechen. Als Materialien für solche inerten Formkörper kommen prinzipiell alle diejenigen in Betracht, die sich auch als Trägermaterial für erfindungsgemäß geeignete Schalenkatalysatoren eignen. Solche Materialien sind insbesondere unpörse Oxide wie Aluminiumoxide, Siliciumdioxid, Thoriumdioxid, Zirkondioxid, Siliciumcarbid, Silikate wie Magnesium- oder Aluminiumsilikat oder Steatit.

Multimetalloxidaktivmassen für Katalysatoren zur Propylen → Acrolein
Partialoxidation mit erhöhter Aktivität enthalten erfindungsgemäß zweckmäßig die Metallelemente Mo, Fe und Bi. Von besonders erhöhter Aktivität sind sie dann, wenn ihre Stöchiometrie folgender Formel I genügt:

Mo₁₂ Biₐ Fe_{b} X¹_{c} X²_{d} X³ₑ ³ₑ X⁴_{f} Oₙ (I),

mit
X¹ = Nickel und/oder Kobalt
   X² = Thallium, ein Alkalimetall und/oder ein Erdalkalimetall,
   X³ = Zink, Phosphor, Arsen, Bor, Antimon, Zinn, Cer, Blei und/oder Wolfram,
   X⁴ = Silicium, Aluminium, Titan und/oder Zirkonium,
a = 0,5 bis 5,
   b = 0,01 bis 5, vorzugsweise 2 bis 4,
   c = 0 bis 10, vorzugsweise 3 bis 10,
   d = 0 bis 2, vorzugsweise 0,02 bis 2,
   e = 0 bis 8, vorzugsweise 0 bis 5,
   f = 0 bis 10 und
   n = eine Zahl, die durch die Wertigkeit und Häufigkeit der von Sauerstoff verschiedenen Elemente in (I) bestimmt wird.

Das Vorgenannte gilt insbesondere dann, wenn die Aktivmasse die bezüglich spezifischer Oberfläche und Porenvolumen bereits angesprochenen Eigenschaften aufweist.

Multimetalloxidaktivmassen für Katalysatoren zur Acrolein → Acrylsäure Partialoxidation mit erhöhter Aktivität enthalten anwendungstechnisch zweckmäßig die Metallelemente Mo und V. Von besonders erhöhter Aktivität sind sie dann, wenn ihre Stöchiometrie folgender Formel II genügt:

Mo₁₂ Vₐ X¹_{b} X²_{c} X³_{d} X⁴ₑ X⁵_{f} X⁶_{g} Oₙ (II),

mit
X¹ = W, Nb, Ta, Cr und/oder Ce,
   X² = Cu, Ni, Co, Fe, Mn und/oder Zn,
   X³ = Sb und/oder Bi,
   X⁴ = eines oder mehrere Alkalimetalle,
   X⁵ = eines oder mehrere Erdalkalimetalle,
   X⁶ = Si, Al, Ti und/oder Zr,
a = 1 bis 6,
   b = 0,2 bis 4,
   c = 0,5 bis 18,
   d = 0 bis 40,
   e = 0 bis 2,
   f = 0 bis 4,
   g = 0 bis 40 und
   n = eine Zahl, die durch die Wertigkeit und Häufigkeit der von Sauerstoff verschiedenen Elemente in (II) bestimmt wird.

Besonders aktive Ausführungsformen innerhalb der aktiven Multimetalloxide (II) sind jene, die von den nachfolgenden Bedeutungen der Variablen der allgemeinen Formel II erfasst werden:
X¹ = W, Nb und/oder Cr,
   X² = Cu, Ni, Co und/oder Fe,
   X³ = Sb,
   X⁴ = Na und/oder K,
   X⁵ = Ca, Sr, und/oder Ba,
   X⁶ = Si, Al und/oder Ti,
a = 1,5 bis 5,
   b = 0,5 bis 2,
   c = 0,5 bis 3,
   d = 0 bis 2,
   e = 0 bis 0,2,
   f = 0 bis 1 und
   n = eine Zahl, die durch die Wertigkeit und Häufigkeit der von Sauerstoff verschiedenen Elemente in II bestimmt wird.

Mit Vorteil werden die partiellen heterogen katalysierten Gasphasenoxidationen einer C₃-Vorläuferverbindung in Rohrbündelreaktoren durchgeführt. Das Katalysatorfestbett befindet sich in den Reaktionsrohren des Rohrbündelreaktors. Die Kontaktrohre werden von einem flüssigen Wärmeträger (in der Regel eine Metallschmelze oder ein flüssiges Metall) umströmt, um die Reaktionswärme abzuführen.

Die Belastung des Katalysator(fest)betts mit Propylen bzw. Acrolein kann in beiden Fällen ≥ 70 Nl/l·h, oder ≥ 90 Nl/l·h, oder ≥ 110 Nl/l·h, oder ≥ 130 Nl/l·h, oder ≥ 140 Nl/l·h, oder ≥ 160 Nl/l·h, oder ≥ 180 Nl/l·h, oder ≥ 240 Nl/l·h, oder ≥ 300 Nl/l·h betragen.
In der Regel beträgt sie jedoch ≤ 600 Nl/l·h (die Belastungswerte sind dabei jeweils auf das Volumen des Katalysatorfestbetts', ausschließlich gegebenenfalls mitverwendeter Abschnitte die ausschließlich aus Inertmaterial bestehen, bezogen).

Unter der Belastung des Katalysator(fest)betts mit Reaktionsgasausgangsgemisch wird in dieser Schrift die Menge an Reaktionsgasausgangsgemisch in Normlitern (Nl; das Volumen in Litern, das die entsprechende Menge an Reaktionsgasausgangsgemisch bei Normalbedingungen, d. h. bei 25° C und 1 bar, einnehmen würde) verstanden, die pro Stunde durch einen Liter Katalysator(fest)bett geführt wird. Die Belastung des Katalysator(fest)betts kann auch nur auf eine Komponente des Reaktionsgasausgangsgemischs bezogen sein. Dann ist sie die Menge dieser Komponente in Normlitern, die als Bestandteil des entsprechenden Reaktionsgasausgangsgemischs pro Stunde durch einen Liter des Katalysator(fest)betts geführt wird.

Als Inertgas werden in dieser Schrift ganz allgemein solche Gase verstanden, die im Verlauf der Partialoxidation, jeweils für sich, zu wenigstens 95 mol-%, bevorzugt zu wenigstens 97 mol-% und ganz besonders bevorzugt zu 99 mol-% oder mehr chemisch unverändert erhalten bleiben.

Die Reaktionstemperatur beträgt bei der Propylen → Acrolein Partialoxidation in der Regel 270 bis 450° C bzw. 280 bis 420° C, vorzugsweise 300 bis 380° C. Die Reaktionstemperatur beträgt bei der Acrolein → Acrylsäure Partialoxidation in der Regel 200 bis 370° C bzw. 200 bis 320° C, vorzugsweise 220 bis 300° C.

Der Arbeitsdruck kann bei den erfindungsgemäßen Partialoxidationen generell sowohl unterhalb von Normaldruck (z. B. bis zu 0,5 atm, das Reaktionsgasgemisch wird durchgesaugt) als auch oberhalb von Normaldruck liegen. Typischerweise wird der Arbeitsdruck bei Werten von 1 bis 5 atm, häufig 1,5 bis 3,5 atm liegen. Normalerweise wird der Arbeitsdruck bei den erfindungsgemäßen Partialoxidationen 100 atm nicht überschreiten.

Als Quelle für den im Rahmen der Partialoxidationen als Oxidationsmittel erforderlichen molekularen Sauerstoff kommt sowohl Luft, als auch zu molekularem Stickstoff entreicherte Luft in Betracht.

Die Auswahl der für die erfindungsgemäße Partialoxidation des Propylen (zu Acrolein) bzw. Acroleins (zu Acrylsäure) zu verwendenden Katalysatoren erfolgt in der Regel so, dass die Selektivität der Zielproduktbildung (Acrolein bzw. Acrylsäure) in der Regel ≥ 83 mol-%, häufig ≥ 85 mol-%, oder ≥ 88 mol-%, oft ≥ 90 mol-%, oder ≥ 93 mol-% oder mehr beträgt.

Typische Reaktionsgasausgangsgemische für die Propylenpartialoxidation (zu Acrolein) können z. B. enthalten:

| | |
|---|---|
| 5 bis 12 Vol.-% | Propylen, |
| 2 bis 15 Vol.-% | Wasser, |
| ≥ 0 bis 10 Vol.-% | Propan, |
| ≥ 0,1 bis 5 Vol.-% | von Propylen, Propan, Wasser, Sauerstoff und Stickstoff verschiedene Bestandteile, |

soviel molekularen Sauerstoff, dass das molare Verhältnis von molekularem Sauerstoff zu Propylen 1 bis 3 beträgt, und als Restmenge bis zu 100 Vol.-% der Gesamtmenge molekularen Stick stoff.

Alternativ kann das Reaktionsgasausgangsgemisch für die Propylen Partialoxidation (zu Acrolein) enthalten:

| | |
|---|---|
| 6 bis 10 Vol.-% | Propylen, |
| 8 bis 18 Vol.-% | molekularer Sauerstoff, |
| 6 bis 30 Vol.-% | Propan und |
| 32 bis 72 Vol.-% | molekularen Stickstoff. |

Reaktionsgasgemische für die Propylen → Acrolein Partialoxidation können auch bis zu 20 Vol.-% H₂ enthalten.
Reaktionsgasausgangsgemische für die Acrolein Partialoxidation (zu Acrylsäure) enthalten z. B.

| | |
|---|---|
| 4 bis 8 Vol.-% | Acrolein, |
| 2 bis 9 Vol.-% | molekularer Sauerstoff, |
| 0 bis 30 Vol.-% | Propan, |
| 30 bis 75 Vol.-% | molekularer Stickstoff und |
| 5 bis 30 Vol.-% | Wasserdampf, |

oder

| | |
|---|---|
| 3 bis 25 Vol.-% | Acrolein, |
| 5 bis 65 Vol.-% | molekularer Sauerstoff, |
| 6 bis 70 Vol.-% | Propan, |
| 0 bis 20 Vol.-% | molekularer Wasserstoff und |
| 5 bis 65 Vol.-% | Wasserdampf. |

Erfindungsgemäß besonders relevant sind solche Partialoxidationen des Propylens (zu Acrolein) bzw. des Acroleins (zu Acrylsäure), bei denen das Produktgasgemisch noch ≥ 0 bis 4 Vol.-% (z. B. bis 0,5 Vol.-%, oder bis 1 Vol.-%, oder bis 1,5 Vol.-%, oder bis 2 Vol.-%, oder bis 2,5 Vol.-%) an molekularem Sauerstoff enthält.

Grundsätzlich können die organischen Gehalte der flüssigen Phase P* an ihren Bestandteilen gaschromatographisch ermittelt werden.

Zur Überführung der im Produktgasgemisch der erfindungsgemäß durchzuführenden Partialoxidation enthaltenen Acrylsäure und Benzoesäure in die flüssige Phase P kommen prinzipiell absorptive und/oder kondensative Maßnahmen in Betracht.

Als Absorptionsmittel kommen dabei z. B. Wasser, wässrige Lösungen (diese können z. B. 0,1 bis 10 Gew.-% Essigsäure, 0,1 bis 5 Gew.-% Acrylsäure und 80 bis 99,8 Gew.-% Wasser enthalten) und/oder organische (insbesondere hydrophobe) Lösungsmittel (z. B. Diphenylether, Diphenyl und/oder Dimethylphthalat) in Betracht. Vorab der Absorption kann das Produktgasgemisch der Partialoxidation anwendungstechnisch zweckmäßig noch einer direkten und/oder indirekten Abkühlung unterworfen werden.

Erfindungsgemäß geeignete Absorptions- und/oder Kondensationsverfahren beschreiben z. B. die Schriften DE-A 10336386, WO 01/96271, DE-A 19631645, DE-A 19501325, EP-A 982289, DE-A 19838845, WO 02/076917, EP-A 1695954, EP-A 695736, EP-A 778225, EP-A 1041062, EP-A 982287, EP-A 982288, US 2004/0242826, EP-A 792867, EP-A 784046, EP-A 695736 , EP-A 1125912, EP-A 1388533 und die in diesen Schriften diesbezüglich zitierte Literatur.

Die Verflüssigung der im Produktgasgemisch enthaltenen Acrylsäure und Benzoesäure kann aber z. B. auch durch Kondensation der höher als Wasser siedenden Bestandteile des Produktgasgemisches erfolgen.

Sowohl die absorptive als auch die kondensative Überführung der Acrylsäure und Benzoesäure in die flüssige Phase P werden ebenso wie deren Überführung in die flüssige Phase P* üblicherweise in trennwirksame Einbauten (zur Vergrößerung der Austauschoberfläche) enthaltenden Trennkolonnen vorgenommen.
Absorption und Kondensation können auch einander überlagernd angewendet werden.

Bezüglich der vorgenannten Absorptions- und/oder Kondensationsverfahren geeignete Trennkolonnen sind insbesondere in der DE-A 10336386, der EP-A 1125912 und der US 2004/0242826 A1 offenbart.

Als trennwirksame Einbauten kommen dabei grundsätzlich alle als trennwirksam bekannten Einbauten in Betracht. D. h., sowohl Böden, wie Glockenböden, Dual-Flow-Böden, oder Ventilböden, Füllkörper wie z. B. Raschig-Ringe, oder Packungen, wie z. B. Sulzer-Packungen, können als trennwirksame Einbauten verwendet werden.

In der Trennkolonne wird das, zuvor gegebenenfalls abgekühlte, Produktgasgemisch der Partialoxidation dabei in der Regel von unten nach oben aufsteigend geführt. Im Rahmen einer absorptiven Kondensation wird das Absorptionsmittel in der Trennkolonne normalerweise von oben nach unten bewegt (geführt).

Die flüssige, Acrylsäure und Benzoesäure enthaltende Phase P* kann der Trennkolonne anwendungstechnisch zweckmäßig aus dem flüssigen Kolonnensumpf oder über flüssigen Seitenabzug unterhalb der Zufuhr des, gegebenenfalls abgekühlten, Produktgasgemischs entnommen werden.

Unter Anwendung wenigstens eines thermischen Trennverfahrens werden nun aus der flüssigen Phase P leichter (bezogen auf Atmosphärendruck) als Benzoesäure und Acrylsäure siedende Bestandteile abgetrennt.
Erfindungsgemäß vorteilhaft wird das wenigstens eine thermische Trennverfahren so angewendet, dass die Leichtsiederabtrennung diejenigen Bestandteile der flüssigen Phase P erfasst, deren Siedetemperatur als Reinsubstanz und bei Atmosphärendruck ≤ der Siedetemperatur von Wasser liegt.

Ein besonders günstiges anzuwendendes thermisches Trennverfahren ist die Strippung mit einem Gas (z. B. Luft, molekularer Stickstoff oder ein anderes Gas).
Dazu wird selbiges durch die flüssige Phase P geführt (anwendungstechnisch vorteilhaft im Gegenstrom in einer trennwirksame Einbauten enthaltenden Trennkolonne) und strippt dabei in dieser enthaltene leichtsiedende Bestandteile beim Durchströmen aus selbiger heraus. Mit Vorteil wird dazu die flüssige Phase P zuvor erwärmt. Zweckmäßig erfolgt die Strippung bei einem unterhalb von Atmosphärendruck liegenden Arbeitsdruck.

Durch letztere Maßnahme wird die Strippung von einer Desorption überlagert. Selbstverständlich kann die Desorption aber auch für sich zur Leichtsiederabtrennung angewendet werden.

Alternativ zur Strippung und/oder Desorption oder auch denselben nachfolgend kann eine rektifikative Leichtsiederabtrennung durchgeführt werden. Insbesondere mit Blick auf eine Fixierung der Trennlinie beim Siedepunkt des Wassers, ist es zweckmäßig, die rektifikative Leichtsiederabtrennung wenigstens teilweise als azeotrope Rektifikation durchzuführen. Als diesbezüglich geeignete Schleppmittel seien beispielsweise Heptan, Dimethylcyclohexan, Ethylcyclohexan, Toluol, Ethylbenzol, Octan, Chlorbenzol, Xylol oder Mischungen (z. B. solche aus 60 Gew.-% Toluol und 40 Gew.-% Heptan) aus denselben genannt. Als alternative Schleppmittel können aber auch Methylisobutylketon oder Isopropylacetat eingesetzt werden.

Weitere geeignet azeotrope Schleppmittel offenbaren die US 2004/0242826, die EP-A 778255, die EP-A 695736 und der in diesen Schriften zitierte Stand der Technik. Üblicherweise werden vorgenannte Rektifikationen ebenfalls vorteilhaft bei unterhalb von Atmosphärendruck liegenden Arbeitsdrucken durchgeführt. Selbstredend kann jedes der vorgenannten thermischen Trennverfahren sowohl für sich, als auch in Kombination mit einem oder mehreren der anderen genannten thermischen Trennverfahren angewendet werden.

Da erfindungsgemäß vorteilhaft lediglich zum Zweck der Leichtsiederabtrennung thermische Trennverfahren angewendet werden, geht mit der erfindungsgemäßen Verfahrensweise als weiterer Vorteil ein besonders geringfügiges Ausmaß an unerwünschter Polymerisatbildung und/oder ein besonders geringer Bedarf an Polymerisationsinhibitoren einher.

Selbstverständlich werden aber auch alle in dieser Schrift aufgeführten Verfahrensschritte polymerisationsinhibiert durchgeführt. Dabei kann wie im aufgeführten Stand der Technik beschrieben vorgegangen werden. Eine herausragende Position unter der Gesamtmenge der verfügbaren Acrylsäureprozeßstabilisatoren nehmen Dibenzo-1,4-thiazin (PTZ), 4-Hydroxy-2,2,6,6-tetramethylpiperidin-1-oxyl (4-OH-TEMPO) und p-Methoxyphenol (MEHQ) ein, die entweder jeweils für sich, oder paarweise, oder als Dreiergemisch Bestandteil der erfindungsgemäß zu behandelnden Gemische, insbesondere der flüssigen Phasen, sein können. Üblicherweise beträgt die Gesamtmenge von in flüssigen, Acrylsäure enthaltenden, Phasen enthaltenen Polymerisationsinhibitoren, bezogen auf die Gesamtmenge an darin enthaltener Acrylsäure, 0,001 bis 2 Gew.-%.

Nach Durchführung der beschriebenen Leichtsiederabtrennungen verbleibt in der Regel eine flüssige Phase P*, die wenigstens 80 Gew.-% Acrylsäure und, bezogen auf die enthaltene Acrylsäuremenge, wenigstens 0,1 Gew.- ‰ Benzoesäure enthält. Bezogen auf die in der flüssigen Phase P* enthaltene Acrylsäure enthält diese mit Vorteil wenigstens 50 Gew.ppm bzw. wenigstens 0,2 Gew.-%, bzw. 0,2 bis 15 Gew.-% Wasser.

Aufgrund unerwünschter Bildung von Acrylsäure-Oligomeren (Michel-Addukten) in den flüssigen, Acrylsäure enthaltenden, Phasen, bei deren sich selbst überlassen, wird die erfindungsgemäße kristallisative Abtrennung möglichst umgehend nach Erzeugung der flüssigen Phase P* angewandt. Durch thermische Rückspaltung der sich ebenfalls in den Mutterlaugen anreichernden Acrylsäure-Oligomeren lässt sich die in diesen enthaltene Acrylsäure rückgewinnen und in eines der zur Gewinnung der flüssigen Phase P und/oder P* angewandten thermischen Trennverfahren rückführen.

In der Regel wird sich an das erfindungsgemäße Verfahren ein Verfahren anschließen, bei dem Acrylsäurekristallisat aufgeschmolzen und in Polymerisate radikalisch einpolymerisiert wird. Häufig wird es sich bei den vorgenannten Polymerisaten um superabsorbierende Polyacrylate handeln.

### Beispiele

Verwendet wurde ein Rührkessel, der ein Innenvolumen von 2 Litern aufwies. Die Geometrie des Rührkessels war zylindrisch, mit einem Innendurchmesser von 110 mm. Der Rührkessel war aus Glas gefertigt. Der Boden des Rührkessels war jedoch doppelwandig aus Edelstahl gefertigt. Die Edelstahlwanddicke betrug 2 mm. Der Abstand der beiden Wände lag bei ca. 1 cm. Der Zwischenraum zwischen den beiden Wänden wurde von einer Mischung bestehend aus 30 Gew.-% Wasser, 40 Gew.-% Methanol und 30 Gew.-% Ethylenglycol als Kühlmedium durchflossen (wenigstens 75 l/h).

Der Inhalt des Rührkessels wurde mittels eines 2-flügeligen Blattrührers gerührt (die Blatthöhe betrug 50 mm, der Durchmesser 57 mm, und die Umdrehungszahl lag bei 500 U/min).

Gefüllt wurde der Rührkessel bei allen Versuche mit 1500 bis 1650 g einer Reinacrylsäure, deren Acrylsäuregehalt ≥ 99,5 Gew.-% betrug und die mit unterschiedlichen Gehalten an Benzoesäure dotiert war (ihre Temperatur betrug 25 °C). Ihr Wassergehalt lag bei 148 Gew.ppm. Darüber hinaus waren die Reinacrylsäuren durch einen Gehalt von ca. 250 - 270 Gew.-ppm Phenothiazin (PTZ) polymerisationsinhibiert. Mit Abkühlgeschwindigkeiten von 15 K/h bis 60 K/h wurde die Temperatur des Kühlmediums (jeweils von einer Starttemperatur von 11 °C ausgehend) kontinuierlich herabgesetzt.

Das Kristallisationsverfahren wurde jeweils unterbrochen, nachdem sich auf dem Kesselboden eine eine Masse M aufweisende Kristallisatschicht von ca. 10 mm Schichtdicke d abgeschieden hatte. Der Rührkessel war drehbar gelagert. Mit Unterbrechung des Kristallisationsverfahrens wurde der Rührkessel auf den Kopf gedreht, um die verbliebene Mutterlauge (Muttersäure) abfließen zu lassen. Anschließend wurden die abgeflossene Mutterlauge und die abgeschiedenen Kristalle jeweils auf ihren Benzoesäuregehalt analysiert sowie aus den Analysenergebnissen der jeweilige Abreicherungskoeffizient A^{BZS} ermittelt.

Die erzielten Ergebnisse zeigt die nachfolgende Tabelle. Sie zeigt zusätzlich die Kristallwachstumsgeschwindigkeit W in mm/min und die Abkühlzeit t in min.

US Provisional Patent Application No. 60/852674, eingereicht am 19.10.2006 ist eingefügt in die vorliegende Anmeldung durch Literaturhinweis.
Im Hinblick auf die obengenannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

**Tabelle**

| | Benzoesäuregehalt (Gew.- ‰) | Gew.-% PTZ | Abkühlgeschwindigkeit (K/h) | d (mm) | M (g) | W (mm/min) | T (min) | A^{BZS} |
|---|---|---|---|---|---|---|---|---|
| Probe A | 1 | 270 | 15 | 12,9 | 161,2 | 0,152 | 85 | 54,1 |
| Probe B | 1,5 | 270 | 30 | 7,2 | 89,9 | 0,206 | 35 | 31,5 |
| Probe C | 1,5 | 270 | 60 | 10,8 | 120,7 | 0,300 | 36 | 30,3 |

| | | | | | | | | |
|---|---|---|---|---|---|---|---|---|
| Allylacrylat, Benzaldehyd, Cumaron und Furfurale weisen als Acrylsäureverunreinigungen ebenfalls regelmäßig Abreicherungskoeffizienten A >>20 auf. Dagegen liegen die Abreicherungskoeffizienten A für Diacrylsäure, Essigsäure und Propionsäure als Acrylsäureverunreinigungen bei Werten < 10. Der hohe gefundene Abreicherungskoeffizient für Benzoesäure überrascht vor diesem Hintergrund. | | | | | | | | |

## Patentansprüche

1. Verfahren zur Trennung von in einem Produktgasgemisch einer partiellen heterogen katalysierten Gasphasenoxidation einer C₃-Vorläuferverbindung der Acrylsäure neben anderen Produktgasgemischbestandteilen als Haupt- und Nebenprodukt enthaltener Acrylsäure und Benzoesäure, bei dem man die Acrylsäure und die Benzoesäure gemeinsam mit anderen leichter und schwerer als Acrylsäure siedenden Bestandteilen des Produktgasgemisches aus dem Produktgasgemisch in eine flüssige Phase P überführt und von der dabei resultierenden flüssigen Phase P unter Anwendung wenigstens eines thermischen Trennverfahrens leichter als Benzoesäure und Acrylsäure siedende Bestandteile unter Verbleib einer flüssigen Phase P*, die wenigstens 80 Gew.-% Acrylsäure und, bezogen auf die enthaltene Acrylsäuremenge, wenigstens 0,1 Gew.-‰ Benzoesäure enthält, abtrennt, **dadurch gekennzeichnet, dass** die Trennung der Benzoesäure von der Acrylsäure kristallisativ aus der flüssigen Phase P* heraus erfolgt, wobei sich die Acrylsäure im gebildeten Kristallisat und die Benzoesäure in der verbliebenen Mutterlauge anreichert.

2. Verfahren nach Anspruch 1, **dadurch gekennzeichnet, dass** die flüssige Phase P* wenigstens 90 Gew.-% Acrylsäure enthält.

3. Verfahren nach Anspruch 1, **dadurch gekennzeichnet, dass** die flüssige Phase P* wenigstens 95 Gew.-% Acrylsäure enthält.

4. Verfahren nach einem der Ansprüche 1 bis 3, **dadurch gekennzeichnet, dass** die flüssige Phase P*, bezogen auf die enthaltene Acrylsäuremenge, wenigstens 0,3 Gew.- ‰ Bezoesäure enthält.

5. Verfahren nach einem der Ansprüche 1 bis 3, **dadurch gekennzeichnet, dass** die flüssige Phase P*, bezogen auf die enthaltene Acrylsäure, wenigstens 0,5 Gew.- ‰ Benzoesäure enthält.

6. Verfahren nach einem der Ansprüche 1 bis 5, **dadurch gekennzeichnet, dass** der Gehalt der flüssigen Phase P* an Benzoesäure wenigstens 50 Gew.-% der in der flüssigen Phase P* enthaltenen Gewichtsmenge an Benzaldehyd beträgt.

7. Verfahren nach einem der Ansprüche 1 bis 5, **dadurch gekennzeichnet, dass** der Gehalt der flüssigen Phase P* an Benzoesäure wenigstens 100 Gew.-% der in der flüssigen Phase P* enthaltenen Gewichtsmenge an Benzaldehyd beträgt.

8. Verfahren nach einem der Ansprüche 1 bis 5, **dadurch gekennzeichnet, dass** der Gehalt der flüssigen Phase P* an Benzoesäure wenigstens 150 Gew.-% der in der flüssigen Phase P* enthaltenen Gewichtsmenge an Benzaldehyd beträgt.

9. Verfahren nach einem der Ansprüche 1 bis 8, **dadurch gekennzeichnet, dass** bei der kristallisativen Abtrennung verbliebene Mutterlauge wenigstens teilweise in wenigstens eines der zur Erzeugung der flüssigen Phase P* aus der flüssigen Phase P angewandten thermischen Trennverfahren und/oder in wenigstens eines der zur gemeinsamen Überführung der im Produktgas der partiellen Gasphasenoxidation enthaltenen Acrylsäure und Benzoesäure in die flüssige Phase P angewandten Verfahren rückgeführt wird.

10. Verfahren nach einem der Ansprüche 1 bis 9, **dadurch gekennzeichnet, dass** die kristallisative Abtrennung der Acrylsäure aus der Phase P* heraus einstufig erfolgt.

11. Verfahren nach einem der Ansprüche 1 bis 9, **dadurch gekennzeichnet, dass** die kristallisative Abtrennung der Acrylsäure aus der Phase P* heraus mehrstufig erfolgt.

12. Verfahren nach einem der Ansprüche 1 bis 11, **dadurch gekennzeichnet, dass** die kristallisative Abtrennung der Acrylsäure durch Schichtkristallisation erfolgt.

13. Verfahren nach einem der Ansprüche 1 bis 12, **dadurch gekennzeichnet, dass** die kristallisative Abtrennung der Acrylsäure durch Suspensionskristallisation erfolgt.

14. Verfahren nach Anspruch 13, **dadurch gekennzeichnet, dass** das gebildete Suspensionskristallisat in einer Waschkolonne von verbliebener Mutterlauge getrennt wird.

15. Verfahren nach Anspruch 14, **dadurch gekennzeichnet, dass** als Waschflüssigkeit die Schmelze von in der Waschkolonne vorab abgetrennten Acrylsäurekristallen verwendet wird.

16. Verfahren nach einem der Ansprüche 1 bis 15, **dadurch gekennzeichnet, dass** es die folgenden Verfahrensschritte umfasst:
a) Auskristallisieren von Acrylsäure aus einer flüssigen Phase P*,
b) Trennen des Acrylsäurekristallisats von der verbliebenen Mutterlauge,
c) wenigstens teilweises Aufschmelzen des abgetrennten Acrylsäurekristallisats und
d) wenigstens teilweises Rückführen des aufgeschmolzenen Acrylsäure-kristallisats zum Schritt b) und/oder zum Schritt a).

17. Verfahren nach einem der Ansprüche 1 bis 16, **dadurch gekennzeichnet, dass** die flüssige Phase P* wenigstens 150 Gew.ppm Wasser enthält.

18. Verfahren nach einem der Ansprüche 1 bis 17, **dadurch gekennzeichnet, dass** die Überführung der im Produktgasgemisch der partiellen Gasphasenoxidation enthaltenen Acrylsäure und Benzoesäure durch Absorption mittels einer wässrigen Lösung erfolgt.

19. Verfahren nach einem der Ansprüche 1 bis 18, **dadurch gekennzeichnet, dass** die C₃-Vorläuferverbindung Propylen ist.

20. Verfahren nach einem der Ansprüche 1 bis 18, **dadurch gekennzeichnet, dass** die C₃-Vorläuferverbindung Propan ist.

21. Verfahren nach einem der Ansprüche 1 bis 18, **dadurch gekennzeichnet, dass** die C₃-Vorläuferverbindung Acrolein ist, und der Acroleinumsatz in der Acrolein-partialoxidation ≥ 99,5 mol-% beträgt.

22. Verfahren nach einem der Ansprüche 1 bis 21, **dadurch gekennzeichnet, dass** sich ein Verfahren, bei dem Acrylsäurekristallisat aufgeschmolzen und in Polymerisate radikalisch einpolymerisiert wird, anschließt.

## Claims

1. A process for separating acrylic acid and benzoic acid present as the main product and by-product as well as other product gas mixture constituents in a product gas mixture of a partial heterogeneously catalyzed gas phase oxidation of a C₃ precursor compound of acrylic acid, in which the acrylic acid and the benzoic acid, together with other constituents of the product gas mixture which have lower and higher boiling points than acrylic acid, are converted from the product gas mixture to a liquid phase P, and constituents having a lower boiling point than benzoic acid and acrylic acid are removed from the resulting liquid phase P using at least one thermal separating process to leave a liquid phase P* which comprises at least 80% by weight of acrylic acid and, based on the amount of acrylic acid present, at least 0.1 per mille by weight of benzoic acid, which comprises separating the benzoic acid from the acrylic acid by crystallization out of the liquid phase P*, the acrylic acid accumulating in the crystals formed and the benzoic acid in the remaining mother liquor.

2. The process according to claim 1, wherein the liquid phase P* comprises at least 90% by weight of acrylic acid.

3. The process according to claim 1, wherein the liquid phase P* comprises at least 95% by weight of acrylic acid.

4. The process according to any of claims 1 to 3, wherein the liquid phase P*, based on the amount of acrylic acid present, comprises at least 0.3 per mille by weight of benzoic acid.

5. The process according to any of claims 1 to 3, wherein the liquid phase P*, based on the amount of acrylic acid present, comprises at least 0.5 per mille by weight of benzoic acid.

6. The process according to any of claims 1 to 5, wherein the content in the liquid phase P* of benzoic acid is at least 50% by weight of the weight of benzaldehyde present in the liquid phase P*.

7. The process according to any of claims 1 to 5, wherein the content in the liquid phase P* of benzoic acid is at least 100% by weight of the weight of benzaldehyde present in the liquid phase P*.

8. The process according to any of claims 1 to 5, wherein the content in the liquid phase P* of benzoic acid is at least 150% by weight of the weight of benzaldehyde present in the liquid phase P*.

9. The process according to any of claims 1 to 8, wherein mother liquor remaining in the crystallizative removal is recycled at least partly into at least one of the thermal separation processes employed to obtain the liquid phase P* from the liquid phase P and/or into at least one of the processes employed for the combined conversion of the acrylic acid and benzoic acid present in the product gas of the partial gas phase oxidation into the liquid phase P.

10. The process according to any of claims 1 to 9, wherein the crystallizative removal of the acrylic acid out of the phase P* is effected in one stage.

11. The process according to any of claims 1 to 9, wherein the crystallizative removal of the acrylic acid out of the phase P* is effected in more than one stage.

12. The process according to any of claims 1 to 11, wherein the crystallizative removal of the acrylic acid is effected by layer crystallization.

13. The process according to any of claims 1 to 12, wherein the crystallizative removal of the acrylic acid is effected by suspension crystallization.

14. The process according to claim 13, wherein the suspension crystals formed are separated from the remaining mother liquor in a wash column.

15. The process according to claim 14, wherein the wash liquid used is the melt of acrylic acid crystals removed beforehand in the wash column.

16. The process according to any of claims 1 to 15, which comprises the following process steps:
a) crystallization of acrylic acid out of a liquid phase P*,
b) separation of the acrylic acid crystals from the remaining mother liquor,
c) at least partial melting of the acrylic acid crystals removed and
d) at least partial recycling of the molten acrylic acid crystals to step b) and/or to step a).

17. The process according to any of claims 1 to 16, wherein the liquid phase P* comprises at least 150 ppm by weight of water.

18. The process according to any of claims 1 to 17, wherein the acrylic acid and benzoic acid present in the product gas mixture of the partial gas phase oxidation are converted by absorption by means of an aqueous solution.

19. The process according to any of claims 1 to 18, wherein the C₃ precursor compound is propylene.

20. The process according to any of claims 1 to 18, wherein the C₃ precursor compound is propane.

21. The process according to any of claims 1 to 18, wherein the C₃ precursor compound is acrolein and the acrolein conversion in the acrolein partial oxidation is ≥ 99.5 mol%.

22. The process according to any of claims 1 to 21, which is followed by a process in which acrylic acid crystals are melted and polymerized free-radically to polymers.

## Revendications

1. Procédé pour la séparation d'acide acrylique et d'acide benzoïque contenus en tant que produit principal et produit secondaire, en plus d'autres composants du mélange de gaz produit, dans un mélange de gaz produit d'une oxydation partielle en phase gazeuse, catalysée par catalyse hétérogène, d'un composé précurseur en C₃ de l'acide acrylique, dans lequel on convertit en une phase liquide P à partir du mélange de gaz produit l'acide acrylique et l'acide benzoïque conjointement avec d'autres composants, à plus bas et plus haut point d'ébullition que l'acide acrylique, du mélange de gaz produit, et on sépare de la phase liquide P qui en résulte, en utilisant au moins un procédé de séparation thermique, les composants à plus bas point d'ébullition que l'acide benzoïque et l'acide acrylique, de sorte qu'il reste une phase liquide P* qui contient au moins 80 % en poids d'acide acrylique et, par rapport à la quantité d'acide acrylique contenue, au moins 0,1 % en poids d'acide benzoïque, **caractérisé en ce que** la séparation de l'acide benzoïque d'avec l'acide acrylique s'effectue par cristallisation à partir de la phase liquide P*, l'acide acrylique s'accumulant dans le cristallisat formé et l'acide benzoïque s'accumulant dans la liqueur mère restante.

2. Procédé selon la revendication 1, **caractérisé en ce que** la phase liquide P* contient au moins 90 % en poids d'acide acrylique.

3. Procédé selon la revendication 1, **caractérisé en ce que** la phase liquide P* contient au moins 95 % en poids d'acide acrylique.

4. Procédé selon l'une quelconque des revendications 1 à 3, **caractérisé en ce que** la phase liquide P* contient au moins 0,3 % en poids d'acide benzoïque par rapport à la quantité d'acide acrylique contenue.

5. Procédé selon l'une quelconque des revendications 1 à 3, **caractérisé en ce que** la phase liquide P* contient au moins 0,5 % en poids d'acide benzoïque par rapport à l'acide acrylique contenu.

6. Procédé selon l'une quelconque des revendications 1 à 5, **caractérisé en ce que** la teneur en acide benzoïque de la phase liquide P* représente d'au moins 50 % en poids de la quantité pondérale de benzaldéhyde contenue dans la phase liquide P*.

7. Procédé selon l'une quelconque des revendications 1 à 5, **caractérisé en ce que** la teneur en acide benzoïque de la phase liquide P* représente au moins 100 % en poids de la quantité pondérale de benzaldéhyde contenue dans la phase liquide P*.

8. Procédé selon l'une quelconque des revendications 1 à 5, **caractérisé en ce que** la teneur en acide benzoïque de la phase liquide P* représente au moins 150 % en poids de la quantité pondérale de benzaldéhyde contenue dans la phase liquide P*.

9. Procédé selon l'une quelconque des revendications 1 à 8, **caractérisé en ce que** la liqueur mère restant lors de la séparation par cristallisation est recyclée au moins en partie dans au moins l'un des procédés de séparation thermique utilisés pour la production de la phase liquide P* à partir de la phase liquide P et/ou dans au moins l'un des procédés utilisés pour la conversion conjointe de l'acide acrylique et de l'acide benzoïque, contenus dans le gaz produit de l'oxydation partielle en phase gazeuse, en la phase liquide P.

10. Procédé selon l'une quelconque des revendications 1 à 9, **caractérisé en ce que** la séparation par cristallisation de l'acide acrylique à partir de la phase P* s'effectue en une seule étape.

11. Procédé selon l'une quelconque des revendications 1 à 9, **caractérisé en ce que** la séparation par cristallisation de l'acide acrylique à partir de la phase P* s'effectue en plusieurs étapes.

12. Procédé selon l'une quelconque des revendications 1 à 11, **caractérisé en ce que** la séparation par cristallisation de l'acide acrylique s'effectue par cristallisation en couches.

13. Procédé selon l'une quelconque des revendications 1 à 12, **caractérisé en ce que** la séparation par cristallisation de l'acide acrylique s'effectue par cristallisation en suspension.

14. Procédé selon la revendication 13, **caractérisé en ce que** le cristallisat en suspension formé est séparé de la liqueur mère restante dans une colonne de lavage.

15. Procédé selon la revendication 14, **caractérisé en ce qu'**on utilise comme liquide de lavage la masse fondue de cristaux d'acide acrylique précédemment séparés dans la colonne de lavage.

16. Procédé selon l'une quelconque des revendications 1 à 15, **caractérisé en ce qu'**il comprend les étapes suivantes de processus :
a) séparation par cristallisation d'acide acrylique à partir d'une phase liquide P*,
b) séparation du cristallisat d'acide acrylique d'avec la liqueur mère restante,
c) fusion au moins partielle du cristallisat d'acide acrylique séparé et
d) recyclage au moins partiel du cristallisat de l'acide acrylique fondu, dans l'étape b) et/ou dans l'étape a).

17. Procédé selon l'une quelconque des revendications 1 à 16, **caractérisé en ce que** la phase liquide P* contient au moins 150 ppm en poids d'eau.

18. Procédé selon l'une quelconque des revendications 1 à 17, **caractérisé en ce que** la conversion de l'acide acrylique et de l'acide benzoïque contenus dans le mélange de produit de l'oxydation partielle en phase gazeuse s'effectue par absorption au moyen d'une solution aqueuse.

19. Procédé selon l'une quelconque des revendications 1 à 18, **caractérisé en ce que** le composé précurseur en C₃ est le propylène.

20. Procédé selon l'une quelconque des revendications 1 à 18, **caractérisé en ce que** le composé précurseur en C₃ est le propane.

21. Procédé selon l'une quelconque des revendications 1 à 18, **caractérisé en ce que** le composé précurseur en C₃ est l'acroléine, et le degré de conversion de l'acroléine dans l'oxydation partielle de l'acroléine est ≥ 99,5 % en moles.

22. Procédé selon l'une quelconque des revendications 1 à 21, **caractérisé en ce qu'**il y fait suite un procédé dans lequel le cristallisat d'acide acrylique est fondu et incorporé par polymérisation radicalaire dans des polymérisats.
